(19) Europäisches Patentamt
European Patent Office
Office européen des brevets

(11) EP 3 507 271 B1

(12) EUROPÄISCHE PATENTSCHRIFT

(45) Veröffentlichungstag und Bekanntmachung des Hinweises auf die Patenterteilung:
**29.09.2021 Patentblatt 2021/39**

(21) Anmeldenummer: **17757558.6**

(22) Anmeldetag: **29.08.2017**

(51) Int Cl.:
***C07C 227/16*** *(2006.01)* ***C07C 229/22*** *(2006.01)*

(86) Internationale Anmeldenummer:
**PCT/EP2017/071608**

(87) Internationale Veröffentlichungsnummer:
**WO 2018/041804 (08.03.2018 Gazette 2018/10)**

(54) **VERFAHREN ZUR HERSTELLUNG VON SALICYLOYL-L-CARNITIN-HYDROCHLORID**

VERFAHREN ZUR HERSTELLUNG VON SALICYLOYL-L-CARNITIN-HYDROCHLORID

PROCÉDÉ POUR LA PRÉPARATION DE CHLORHYDRATE DE SALICYLOYL-L-CARNITINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.08.2016 EP 16186393**

(43) Veröffentlichungstag der Anmeldung:
**10.07.2019 Patentblatt 2019/28**

(73) Patentinhaber: **Drug'On Pharma Switzerland AG**
**4410 Liestal (CH)**

(72) Erfinder:
- **MEUL, Thomas**
**3930 Visp (CH)**
- **WEBER, Peter**
**4127 Birsfelden (CH)**

(74) Vertreter: **Latscha Schöllhorn Partner AG**
**Grellingerstrasse 60**
**4052 Basel (CH)**

(56) Entgegenhaltungen:
**EP-A1- 0 553 385**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 507 271 B1

**Beschreibung**

GEBIET DER ERFINDUNG

Die vorliegende Erfindung betrifft insbesondere ein Verfahren zur Herstellung von 3-(2-hydroxybenzoyloxy)-4-(trimethylammonio)-buttersäurebetain-Hydrochlorid der Formel (I).

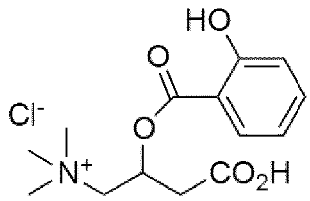

(I)

HINTERGRUND DER ERFINDUNG

3-(2-hydroxybenzoyloxy)-4-(trimethylammonio)-buttersäurebetain-Hydrochlorid ist als Ester der Salicylsäure mit Carnitin (Salicyloylcarnitin) ein Salicylsäurederivat mit vielversprechenden therapeutischen Eigenschaften (EP 553385). Das einzige bekannte Verfahren zur Herstellung von Salicyloylcarnitin geht von L-Carnitin-HCl und dem Säurechlorid der Salicylsäure aus, deren Hydroxy-Gruppe durch eine Methylgruppe geschützt ist. Die Abspaltung dieser Methylgruppe gelingt nur mit hochkonzentrierter Bromwasserstoffsäure. Die Ausbeute dieser Abspaltung ist bescheiden (50%). Zusätzlich bildet sich das Hydrobromid des Salicyloylcarnitins, das für die Verwendung zu pharmazeutischen Zwecken mittels Ionenaustauscherharz entweder in das freie Betain oder in dessen Hydrochlorid überführt werden muss. Über alle Stufen beträgt die Ausbeute an Salicyloylcarnitin-Hydrochlorid lediglich 35%.

Eine Aufgabe der vorliegenden Erfindung ist es daher, ein verbessertes Verfahren zur Herstellung von 3-(2-hydroxybenzoyloxy)-4-(trimethylammonio)-buttersäurebetain-Hydrochlorid bereitzustellen. Insbesondere ist es eine Aufgabe der vorliegenden Erfindung ein kurzes und damit kostengünstiges Verfahren, bevorzugt mit hoher Ausbeute, zur Herstellung von 3-(2-hydroxybenzoyloxy)-4-(trimethylammonio)-buttersäurebetain-Hydrochlorid bereitzustellten.

ZUSAMMENFASSUNG DER ERFINDUNG

Es wurde überraschenderweise gefunden, dass die Umsetzung von Carnitin oder Salzen davon, insbesondere von L-Carnitin Hydrochlorid, mit 2-(Alkoxy)benzoylchloriden, deren Alkoxygruppen unter Einwirkung von Chlorwasserstoffsäure zur entsprechenden Hydroxygruppe spaltbar sind, wie insbesondere und vorzugsweise 2-(Benzyloxy)benzoylchlorid, 2-(Isopropoxy)benzoylchlorid oder 2-(Isobutoxy)benzoylchlorid, in einem Eintopfverfahren und in hoher Ausbeute 3-(2-hydroxybenzoyloxy)-4-(trimethylammonio)-buttersäurebetain Hydrochlorid der Formel (I) liefert.

Es wurde darüber hinaus überraschenderweise gefunden, dass die Ausbeute des erfindungsgemässen Verfahrens weiter erhöht werden kann, wenn die Umsetzung zur Bildung der im Eintopfverfahren zwischenzeitlich entstehenden 2-(Alkoxy)benzoylcarnitin-Derivate der Formel (II) durch vorzugsweise Anlegen eines Vakuums oder Durchleiten eines Inertgases gefördert wird, wodurch die entstehende Chlorwasserstoffsäure entfernt wird. Das 3-(2-hydroxybenzoyloxy)-4-(trimethylammonio)-buttersäurebetain-Hydrochlorid der Formel (I) wird dann vorzugsweise durch nachträgliches Einleiten von Chlorwasserstoff-Gas gebildet. So erlaubt das erfindungsgemässe Verfahren gar die Reaktion auf der Zwischenstufe der entstehenden 2-(Alkoxy)benzoylcarnitin-Derivate der Formel (II) anzuhalten und die 2-(Alkoxy)benzoylcarnitin-Derivate der Formel (II) zu isolieren.

In einem ersten Aspekt der Erfindung wird ein Verfahren zur Herstellung von 3-(2-hydroxybenzoyloxy)-4-(trimethylammonio)-buttersäurebetain-Hydrochlorid der Formel (I), bereitgestellt, wobei das Verfahren den Schritt umfasst (i) Umsetzung von 3-Hydroxy-4-(trimethylammonio)-buttersäurebetain oder einem Salz davon, wobei vorzugsweise das Salz des 3-Hydroxy-4-(trimethylammonio)-buttersäurebetains das 3-Hydroxy-4-(trimethylammonio)-buttersäurebetain-Hydrochlorid ist, mit einem 2-Alkoxybenzoylchlorid der Formel (III),

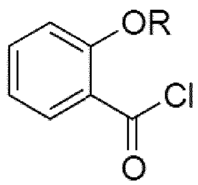

(III)

wobei R ausgewählt ist aus Benzyl und verzweigten C3-C6-Alkylgruppen.

In einem weiteren Aspekt der Erfindung wird eine Verbindung der Formel (II), oder ein Salz davon, bereitgestellt,

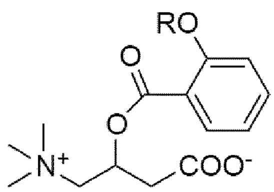

(II)

wobei R ausgewählt ist aus Benzyl und verzweigten C3-C6-Alkylgruppen.

Weitere Aspekte und Ausführungsformen der Erfindung werden im Verlauf dieser Beschreibung offenkundig.

DETAILLIERTE BESCHREIBUNG DER ERFINDUNG

Der Begriff "Carnitin" bezieht sich auf die chemische Verbindung 3-Carboxy-2-hydroxypropyl-N,N,N-trimethylammoniumhydroxid. Die Begriff "3-Hydroxy-4-(trimethylammonio)-buttersäurebetain", "Carnitin" und "3-Carboxy-2-hydroxypropyl-N,N,N-trimethylammoniumhydroxid" werden synonym verwendet. Die Verbindung weist ein asymmetrisches Kohlenstoffatom auf und kann daher in zwei spiegelbildlichen optisch aktiven Formen und als racemisches Gemisch auftreten. Bevorzugt ist "L-Carnitin" (L-(-)-Carnitin) und somit das (R)-Enantiomer (R)-(3-Carboxy-2-hydroxypropyl)-N,N,N-trimethylammonium-hydroxid.

Der Begriff "Betain" bezieht sich auf eine chemische Verbindung, die in ihrer Molekülstruktur sowohl eine positive als auch eine negative Ladung trägt, nach außen hin also ungeladen ist. In Betainen können sich diese Ladungen, anders als bei Zwitterionen nicht durch Protonenwanderung ausgleichen. Ein Beispiel für ein Betain ist Carnitin.

Der Begriff "Inertgas" bezieht sich auf ein Gas, welches unter den gegebenen Reaktionsbedingungen weder mit den Startmaterialien oder Endprodukten, noch den Reagenzien, Zwischenprodukten und Lösungsmitteln chemische Reaktionen eingeht. Typische Inertgase sind Argon und Stickstoff, insbesondere Stickstoff.

Die Begriffe "reduzierter Druck" und "Vakuum" werden in dieser Beschreibung synonym verwendet und beziehen sich auf einen Druck im Inneren des Reaktors, in dem das erfindungsgemässe Verfahren durchgeführt wird, der gegenüber dem vorherrschenden atmosphärischen Druck reduziert ist, zum Beispiel ein Druck von 100-200 mbar.

Der Begriff "Überdruck" bezieht sich auf einen Druck im Inneren des Reaktors, in dem das erfindungsgemässe Verfahren durchgeführt wird, der gegenüber dem vorherrschenden atmosphärischen Druck erhöht ist, beispielsweise ein Druck von 2 bar.

Der Begriff "C1-C6-Carbonsäure", wie hier verwendet, bezieht sich auf eine organische Verbindung mit einer Kette von einem bis 6 Kohlenstoffatomen, die eine oder mehrere Carboxygruppen (-COOH) trägt. Vorzugsweise, bezieht sich der Begriff "C1-C6-Carbonsäure", wie hier verwendet, auf eine Mono-Carbonsäure, also auf eine organische Verbindung mit einer Kette von einem bis 6 Kohlenstoffatomen, die eine Carboxygruppe trägt. Typisch und vorzugsweise bezieht sich die Kette von einem bis 6 Kohlenstoffatomen auf eine lineare oder verzweigte, bevorzugt lineare Kette von Kohlenstoffatomen, die wahlweise substituiert sein kann. Typische und bevorzugte Substitutionen sind insbesondere halogenierte, bevorzugt chlorierte, C1-C6-Carbonsäuren. Sehr bevorzugte "C1-C6-Carbonsäuren" für die vorliegende Erfindung sind Monocarbonsäuren mit einer linearen oder verzweigten, bevorzugt linearen, gesättigten Kette von Kohlenstoffatomen, die wahlweise substituiert sein kann, besonders bevorzugt hierbei sind Monocarbonsäuren mit einer linearen, gesättigten Kette von Kohlenstoffatomen, die vorzugsweise substituiert sein kann. Typische und bevorzugte Substitutionen dieser "Cl-C6-Carbonsäure" sind insbesondere halogenierte, bevorzugt chlorierte, Monocarbonsäuren mit einer linearen, gesättigten Kette von Kohlenstoffatomen.

In einem ersten Aspekt der Erfindung wird ein Verfahren zur Herstellung von 3-(2-hydroxybenzoyloxy)-4-(trimethylammonio)-buttersäurebetain-Hydrochlorid der Formel (I), bereitgestellt, wobei das Verfahren den Schritt umfasst (i) Umsetzung von 3-Hydroxy-4-(trimethylammonio)-buttersäurebetain oder einem Salz davon, wobei vorzugsweise das Salz des 3-Hydroxy-4-(trimethylammonio)-buttersäurebetains das 3-Hydroxy-4-(trimethylammonio)-buttersäurebetain-Hydrochlorid ist, mit einem 2-Alkoxybenzoylchlorid der Formel (III),

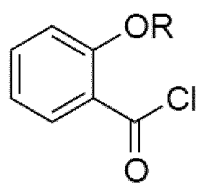
(III)

wobei R ausgewählt ist aus Benzyl und verzweigten C3-C6-Alkylgruppen. Ein Vorteil des erfindungsgemässen Verfahrens ist es daher insbesondere, dass das erfindungsgemässe Verfahren einstufig und als Eintopfreaktion durchgeführt werden kann, was das Verfahren ausserordentlich effizient und kostengünstiger macht.

Das erfindungsgemässe Verfahren eignet sich selbstverständlich, je nach Ausgangsmaterial, gleichermassen für die Herstellung von racemischem wie von optisch aktivem 3-(2-hydroxybenzoyloxy)-4-(trimethylammonio)-buttersäurebetain-Hydrochlorid. Ganz besonders bevorzugt wird im erfindungsgemässen Verfahren das (R)-(-)-3-(2-Hydroxybenzoyloxy)-4-trimethylammonio)-buttersäure-Hydrochlorid der Formel (Ia), ausgehend von (L)-Carnitin oder einem Salz davon, insbesondere ausgehend von (L)-Carnitin Hydrochlorid.

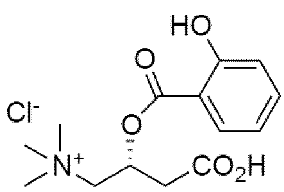
(Ia)

Daher wird in einem weiteren Aspekt der Erfindung ein Verfahren zur Herstellung von (R)-(-)-3-(2-hydroxybenzoyloxy)-4-(trimethylammonio)-buttersäurebetain-Hydrochlorid der Formel (Ia), bereitgestellt, dadurch gekennzeichnet, dass (R)-(3-Hydroxy-4-(trimethylammonio))-buttersäurebetain ((L)-Carnitin) oder ein Salz davon, vorzugsweise das Hydrochlorid, mit einem 2-Alkoxybenzoylchlorid der Formel (III) (2-Alkoxy-Salicyloylchlorid) umgesetzt wird, wobei R ausgewählt ist aus benzyl und verzweigten C3-C6-Alkylgruppen.

In einer Ausführungsform, ist das Verhältnis von besagtem 3-Hydroxy-4-(trimethyl-ammonio)-buttersäurebetain, oder einem Salz davon, zu besagtem 2-Alkoxylbenzoylchlorid der Formel (III) 1:1 bis 1: 2.5 (Mol: Mol).

In einer weiteren Ausführungsform des erfindungsgemässen Verfahrens erfolgt die besagte Umsetzung mit 3-Hydroxy-4-(trimethylammonio)-buttersäurebetain und besagtem 2-Alkoxybenzoylchlorid der Formel (III). In einer weiteren Ausführungsform des erfindungsgemässen Verfahrens erfolgt die besagte Umsetzung mit einem Salz von 3-Hydroxy-4-(trimethylammonio)-buttersäurebetain, bevorzugt mit 3-Hydroxy-4-(trimethylammonio)-buttersäurebetain-Hydrochlorid oder 3-Hydroxy-4-(trimethylammonio)-buttersäurebetain Tartrat, besonders bevorzugt mit 3-Hydroxy-4-(trimethylammonio)-buttersäurebetain-Hydrochlorid und besagtem 2-Alkoxybenzoylchlorid der Formel (III).

In einer weiteren besonders bevorzugten Ausführungsform des erfindungsgemässen Verfahrens umfasst die besagte Umsetzung (i) das (1) Anlegen eines Vakuums; oder das (2) Durchleiten eines Inertgases; und wobei das Verfahren weiter den Schritt (ii) umfasst: das Versetzen des Reaktionsgemisches der besagten Umsetzung (i) mit Chlorwasserstoffsäure. Besonders bevorzugt erfolgt hierbei das besagte Versetzen des Reaktionsgemisches mit Chlorwasserstoffsäure durch Einleiten von gasförmiger Chlorwasserstoffsäure.

Durch diese besonders bevorzugte Ausführungsform des erfindungsgemässen Verfahrens wird die Ausbeute überrascherweise weiter erhöht. Durch Anlegen eines Vakuums oder Durchleiten eines Inertgases, vorzugsweise durch Anlegen eines Vakuums, wird bei der Umsetzung durch die Entfernung der entstehenden Chlorwasserstoffsäure die Bildung der im Eintopfverfahren zwischenzeitlich entstehenden 2-(Alkoxy)benzoylcarnitin-Derivate gefördert. Die *in situ* gebildeten die 2-(Alkoxy)benzoylcarnitin-Derivate der Formel (II) werden dann durch nachträgliches Einleiten von Chlorwasserstoff-Gas zum 3-(2-hydroxybenzoyloxy)-4-(trimethylammonio)-buttersäurebetain-Hydrochlorid der Formel (I) umgesetzt. In einer weiteren besonders bevorzugten Ausführungsform der vorliegenden Erfindung umfasst die besagte Umsetzung (i) das Anlegen eines Vakuums und erfolgt das besagte Versetzen des Reaktionsgemisches mit Chlorwasserstoffsäure (ii) durch Einleiten von gasförmiger Chlorwasserstoffsäure. Typisch und bevorzugt wird das Anlegen eines Vakuums oder Durchleiten eines Inertgases zur Entfernung der entstehenden Chlorwasserstoffsäure kontinuierlich erfolgen. Weiter typisch und bevorzugt, wird die besagte Umsetzung (i) verfolgt, typisch und bevorzugt durch Dünnschicht-Chromatographie. In einer weiteren bevorzugten Ausführungsform erfolgt das besagte Versetzen des Reaktionsgemisches der besagten Umsetzung (i) mit Chlorwasserstoffsäure, bevorzugt das besagte Versetzen des Reaktionsgemisches mit Chlorwasserstoffsäure durch Einleiten von gasförmiger Chlorwasserstoffsäure, nach 70%-iger, bevorzugt 80%-iger, besonders bevorzugt 90%-iger und damit nahezu vollständiger Umsetzung, und weiter besonders bevorzugt vollständiger Umsetzung von 3-Hydroxy-4-(trimethylammonio)-buttersäurebetain oder einem Salz davon, vorzugsweise das Hydrochlorid davon, mit besagtem 2-Alkoxybenzoylchlorid der Formel (III) zum besagten Zwischenprodukt 2-(Alkoxy)benzoylcarnitin-Derivate der Formel (II). Die Bestimmung der Quantifizierung des Verlaufs der besagten Umsetzung (i) liegt im fachmännischen Können.

In einer weiteren besonders bevorzugten Ausführungsform umfasst das besagte Versetzen des Reaktionsgemisches mit Chlorwasserstoffsäure (ii) die Zugabe von Wasser.

Das erfindungsgemässe Verfahren erlaubt gar die Reaktion auf der Zwischenstufe der entstehenden 2-(Alkoxy)benzoylcarnitin-Derivate der Formel (II) anzuhalten und die 2-(Alkoxy)benzoylcarnitin-Derivate der Formel (II) zu isolieren.

Daher wird in einem weiteren Aspekt der Erfindung ein Verfahren zur Herstellung einer Verbindung der Formel (II), oder ein Salz davon, bereitgestellt,

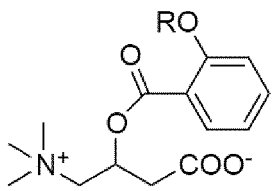

(II)

wobei das Verfahren den Schritt (i) der Umsetzung von 3-Hydroxy-4-(trimethylammonio)-buttersäurebetain oder einem Salz davon, wobei vorzugsweise das Salz des 3-Hydroxy-4-(trimethylammonio)-buttersäurebetains das 3-Hydroxy-4-(trimethylammonio)-buttersäurebetain-Hydrochlorid ist, mit einem 2-Alkoxybenzoylchlorid der Formel (III) umfasst,

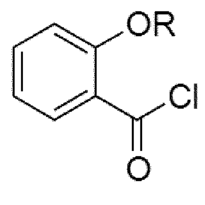

(III)

wobei R ausgewählt ist aus Benzyl und verzweigten C3-C6-Alkylgruppen; und wobei die besagte Umsetzung (i), typisch und bevorzugt zur Entfernung des gebildeten HCl, das (1) Anlegen eines Vakuums; oder (2) das Durchleiten eines Inertgases, umfasst.

Des Weiteren wird in einem weiteren Aspekt der Erfindung eine Verbindung der Formel (II), oder ein

Salz davon, bereitgestellt,

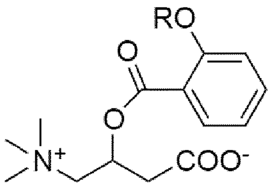

(II)

wobei R ausgewählt ist aus Benzyl und verzweigten C3-C6-Alkylgruppen.

Das Isolieren der entstehenden 2-(Alkoxy)benzoylcarnitin-Derivate der Formel (II) führt zu einer weiteren Ausführungsform der vorliegenden Erfindung bei der nachgängig die isolierten 2-(Alkoxy)benzoylcarnitin-Derivate der Formel (II) mit Chlorwasserstoffsäure versetzt werden. So umfasst in einer weiteren Ausführungsform der vorliegenden Erfindung die besagte Umsetzung (i) das Isolieren eines Zwischenproduktes der allgemeinen Formel (II), oder eines Salzes davon,

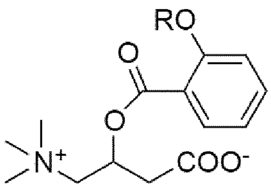

(II)

und ist das besagte Versetzen des Reaktionsgemisches mit Chlorwasserstoffsäure (ii) das Versetzen des besagten Zwischenproduktes der allgemeinen Formel (II) oder eines Salzes davon mit Chlorwasserstoffsäure. In einer bevorzugten Ausführungsform erfolgt das besagte Versetzen des Reaktionsgemisches mit Chlorwasserstoffsäure durch Einleiten von gasförmiger Chlorwasserstoffsäure.

Das erfindungsgemässe Verfahren muss also nicht zwingend als Eintopfverfahren durchgeführt werden, was eine gewisse Flexibilität in der Planung erlaubt.

In einer weiteren bevorzugten Ausführungsform umfasst die besagte Umsetzung (i) das (1) Anlegen eines Vakuums, und wobei das Vakuum 50 mbar bis 800 mbar beträgt. In einer weiteren bevorzugten Ausführungsform beträgt das Vakuum 50 mbar bis 700 mbar, bevorzugt 50 mbar bis 600 mbar, weiter bevorzugt 50 mbar bis 500 mbar, weiter bevorzugt 50 mbar bis 400 mbar, weiter bevorzugt 50 mbar bis 300 mbar, weiter bevorzugt 50 mbar bis 200 mbar, besonders bevorzugt 100 mbar bis 200 mbar, beispielsweise 150 mbar.

In einer weiteren Ausführungsform, wird die besagte Umsetzung (i) in einem Lösungsmittel durchgeführt, wobei das Lösungsmittel ein polares, gegenüber Chlorwasserstoffsäure inertes Lösungsmittel ist. In einer weiteren Ausführungsform, wird das besagte Versetzen des Reaktionsgemisches mit Chlorwasserstoffsäure (ii) in einem Lösungsmittel durchgeführt wird, wobei das Lösungsmittel ein polares, gegenüber Chlorwasserstoffsäure inertes Lösungsmittel ist.

In einer weiteren Ausführungsform, wird die besagte Umsetzung (i) in einem Lösungsmittel durchgeführt, wobei das Lösungsmittel ein protisches, polares und gegenüber Chlorwasserstoffsäure inertes Lösungsmittel ist. In einer weiteren Ausführungsform, wird das besagte Versetzen des Reaktionsgemisches mit Chlorwasserstoffsäure (ii) in einem Lösungsmittel durchgeführt, wobei das Lösungsmittel ein protisches, polares und gegenüber Chlorwasserstoffsäure inertes Lösungsmittel ist.

In einer weiteren bevorzugten Ausführungsform, wird die besagte Umsetzung (i) und das besagte Versetzen des Reaktionsgemisches mit Chlorwasserstoffsäure (ii) in einem Lösungsmittel durchgeführt, wobei das Lösungsmittel ein protisches, polares und gegenüber Chlorwasserstoffsäure inertes Lösungsmittel ist,

In einer weiteren bevorzugten Ausführungsform wird die besagte Umsetzung (i) und das besagte Versetzen des Reaktionsgemisches mit Chlorwasserstoffsäure (ii) in einem Lösungsmittel durchgeführt, wobei das Lösungsmittel aus aliphatischen C1-C6-Carbonsäuren, halogenierten aliphatischen C1-C6-Carbonsäuren und Gemischen davon ausgewählt ist. In einer weiteren bevorzugten Ausführungsform ist das besagte protische, polare und gegenüber Chlorwasserstoffsäure inerte Lösungsmittel ausgewählt aus halogenierten C1-C6-Carbonsäuren und Gemischen davon.

In einer weiteren besonders bevorzugten Ausführungsform wird die besagte Umsetzung (i) und das besagte Versetzen des Reaktionsgemisches mit Chlorwasserstoffsäure (ii) in einem Lösungsmittel durchgeführt, wobei das Lösungsmittel aus halogenierten C1-C6-Carbonsäuren und Gemischen davon ausgewählt ist. In einer weiteren besonders bevorzugten Ausführungsform ist das Lösungsmittel ausgewählt aus halogenierten Essigsäuren und Gemischen davon. In einer weiteren besonders bevorzugten Ausführungsform wird die besagte Umsetzung (i) und das besagte Versetzen des Reaktionsgemisches mit Chlorwasserstoffsäure (ii) in einem Lösungsmittel durchgeführt, wobei das Lösungsmittel aus Monochloressigsäure, Dichloressigsäure und Trichloressigsäure oder Gemischen davon ausgewählt ist. In einer weiteren besonders bevorzugten Ausführungsform wird die besagte Umsetzung (i) und das besagte Versetzen des Reaktionsgemisches mit Chlorwasserstoffsäure (ii) in einem Lösungsmittel durchgeführt, wobei das Lösungsmittel aus Monochloressigsäure, Dichloressigsäure und Trichloressigsäure ausgewählt ist, wobei vorzugsweise das Lösungsmittel Monochloressigsäure oder Dichloressigsäure ist, und wobei besonders bevorzugt das Lösungsmittel Dichloressigsäure ist.

In einer weiteren besonders bevorzugten Ausführungsform wird die besagte Umsetzung (i) und das

besagte Versetzen des Reaktionsgemisches mit Chlorwasserstoffsäure (ii) in einem Lösungsmittel durchgeführt, wobei das Lösungsmittel ein Gemisch aus Monochloressigsäure, Dichloressigsäure und Trichloressigsäure ist. In einer ganz besonders bevorzugten Ausführungsform wird die besagte Umsetzung (i) und das besagte Versetzen des Reaktionsgemisches mit Chlorwasserstoffsäure (ii) in einem Lösungsmittel durchgeführt, wobei das Lösungsmittel ein Gemisch aus Monochloressigsäure und Dichloressigsäure ist.

In einer weiteren besonders bevorzugten Ausführungsform ist R ausgewählt aus Benzyl und sekundären C3-C6-Alkylgruppen.

In einer besonders bevorzugten Ausführungsform ist R aus Benzyl, und sekundären C3-C6-Alkylgruppen ausgewählt, wobei weiter bevorzugt die sekundäre C3-C6-Alkylgruppe aus iso-Propyl, sec-Butyl, 2-Pentyl (sec-Pentyl), 3-Pentyl, 3-Methylbut-2-yl, 2-hexyl, 3-hexyl, 3-methyl-2-pentyl, 4-methyl-2-pentyl und 3,3-dimethyl-2-butyl ausgewählt ist, und weiter bevorzugt die sekundäre C3-C6-Alkylgruppe aus iso-Propyl und sec-Butyl ausgewählt ist, und wobei weiter besonders bevorzugt die sekundäre C3-C6-Alkylgruppe iso-Propyl ist.

In einer besonders bevorzugten Ausführungsform ist R aus Benzyl, iso-Propyl, sec-Butyl, und 2-Pentyl (sec-Pentyl) ausgewählt. In einer weiteren besonders bevorzugten Ausführungsform ist R aus Benzyl, iso-Propyl und sec-Butyl (2-Butyl) ausgewählt. In einer weiteren besonders bevorzugten Ausführungsform ist R Benzyl. In einer weiteren besonders bevorzugten Ausführungsform ist R iso-Propyl. In einer weiteren besonders bevorzugten Ausführungsform ist R sec-Butyl (2-Butyl).

In einer weiteren bevorzugten Ausführungsform wird die besagte Umsetzung (i) bei einer Temperatur von -20°C bis +30°C durchgeführt wird. In einer weiteren bevorzugten Ausführungsform wird die besagte Umsetzung (i) bei einer Temperatur von -20°C bis 10°C, -20°C bis 0°C oder -10°C bis 0°C bevorzugt bei einer Temperatur von -10°C bis 0°C durchgeführt.

In einer weiteren bevorzugten Ausführungsform wird das besagte Versetzen des Reaktionsgemisches mit Chlorwasserstoffsäure (ii), vorzugsweise das Einleiten von gasförmiger Chlorwasserstoffsäure, bei einer Temperatur von 20°C bis 100°C durchgeführt wird. In einer weiteren bevorzugten Ausführungsform wird das besagte Versetzen des Reaktionsgemisches mit Chlorwasserstoffsäure (ii), vorzugsweise das Einleiten von gasförmiger Chlorwasserstoffsäure, bei einer Temperatur von 20°C bis 100°C, 20°C bis 90°C, 20°C bis 80°C, 20°C bis 60°C, 20°C bis 50°C oder 20°C bis 40°C, bevorzugt bei einer Temperatur von 20°C bis 50°C (z.B. 25°C bis 45°C) durchgeführt.

In einer weiteren bevorzugten Ausführungsform wird die besagte Umsetzung (i) und das besagte Versetzen des Reaktionsgemisches mit Chlorwasserstoffsäure (ii), vorzugsweise das Einleiten von gasförmiger Chlorwasserstoffsäure, bei Raumtemperatur durchgeführt.

In einer weiteren bevorzugten Ausführungsform wird das besagte Versetzen des Reaktionsgemisches mit Chlorwasserstoffsäure (ii), vorzugsweise das Einleiten von gasförmiger Chlorwasserstoffsäure, unter einem Überdruck gemessen am Umgebungsdruck durchgeführt wird. In einer Ausführungsform beträgt besagter Überdruck 0.1 bis 5 bar, vorzugsweise 0.1 bis 3 bar, weiter bevorzugt 0.1 bis 2 bar. In einer weiteren besonders bevorzugten Ausführungsform beträgt besagter Überdruck 0.1 bis 1 bar.

In einer bevorzugten Ausführungsform wird das besagte Versetzen des Reaktionsgemisches mit Chlorwasserstoffsäure (ii), vorzugsweise das Einleiten von gasförmiger Chlorwasserstoffsäure, und weiter vorzugsweise die besagte Umsetzung (i), und besonders vorzugsweise das erfindungsgemässe Verfahren, in einem Autoklaven unter einem Überdruck gemessen am Umgebungsdruck durchgeführt.

In einer typischen und bevorzugten Ausführungsform wird besagter Überdruck durch die Umsetzung (i) oder durch das besagte Versetzen des Reaktionsgemisches mit Chlorwasserstoffsäure (ii), vorzugsweise durch die eingeleitete gasförmige Chlorwasserstoffsäure, erzeugt.

In einer weiteren besonders bevorzugten Ausführungsform umfasst das Verfahren weiter den Schritt (iii) das Isolieren des 3-(2-hydroxybenzoyloxy)-4-(trimethylammonio)-buttersäurebetain-Hydrochlorid der Formel (I), wobei das Isolieren durch Kristallisation durch Zugabe eines unpolaren Lösungsmittels erfolgt.

In einer weiteren besonders bevorzugten Ausführungsform umfasst das erfindungsgemässe Verfahren eine Kristallisation des Produkts 3-(2-hydroxybenzoyloxy)-4-(trimethylammonio)-buttersäurebetain-Hydrochlorid direkt aus dem Reaktionsgemisch durch Zugabe eines unpolaren Lösungsmittels.

In einer weiteren bevorzugten Ausführungsform weist das besagte unpolare Lösungsmittel eine Polarität gemäss $E_T(30)$-Skala zwischen 125 kJ/mol und 165 kJ/mol auf (Alan R. Katritzky, Dan C. Fara, Hongfang Yang, Kaido Tämm et al.: Quantitative Measures of Solvent Polarity, S.183 Spectroscopic Measurements). In einer besonders bevorzugten Ausführungsform ist das besagte unpolare Lösungsmittel aus Essigsäureethylester, Diethylether und tert-Butylmethylether ausgewählt. In einer weiteren besonders bevorzugten Ausführungsform ist das besagte unpolare Lösungsmittel Essigsäureethylester.

In einer besonders bevorzugten Ausführungsform des erfindungsgemässen Verfahrens erfolgt die besagte Umsetzung (i) mit (R)-(-)-3-Hydroxy-4-(trimethylammonio)-buttersäurebetain Hydrochlorid und besagtem 2-Alkoxybenzoylchlorid der Formel (III). Typisch und vorzugsweise führt diese besonders bevorzugte Ausführungsform des erfindungsgemässen Verfahrens zur Herstellung von (R)-(-)-3-(2-Hydroxybenzoyloxy)-4-trimethylammonio)-buttersäure-Hydrochlorid der Formel (Ia),

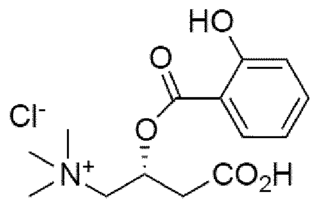

(Ia)

Wie erwähnt, wird in einem weiteren Aspekt der Erfindung eine Verbindung der Formel (II), oder ein Salz davon, bereitgestellt,

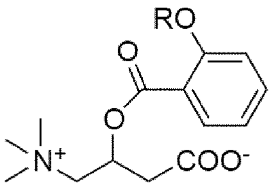

(II)

wobei R ausgewählt ist aus Benzyl und verzweigten C3-C6-Alkylgruppen.

In einer bevorzugten Ausführungsform ist die erfindungsgemässe Verbindung der Formel (II) ausgewählt aus 3-(2-(Butan-2-yloxy)benzoyloxy)-4-trimethylammonio)-buttersäurebetain; 3-(2-Isopropoxybenzoyloxy)-4-trimethylammonio)-buttersäurebetain und 3-(2-benzyloxybenzoyloxy)-4-trimethylammonio)-buttersäurebetain und Salzen davon. In einer besonders bevorzugten Ausführungsform wird eine Verbindung der Formel (II) bereitgestellt, wobei R ausgewählt ist aus benzyl, iso-propyl und sec-butyl. In einer weiteren besonders bevorzugten Ausführungsform wird eine Verbindung der Formel (II) bereitgestellt, wobei die Verbindung der Formel (II) ausgewähhlt ist aus 3-(2-(Butan-2-yloxy)benzoyloxy)-4-trimethylammonio)-buttersäurebetain; 3-(2-Isopropoxybenzoyloxy)-4-trimethylammonio)-buttersäurebetain; 3-(2-benzyloxybenzoyloxy)-4-trimethylammonio)-buttersäurebetain; 3-(2-(Butan-2-yloxy)benzoyloxy)-4-trimethylammonio)-buttersäurebetain Hydrochlorid; 3-(2-Isopropoxybenzoyloxy)-4-trimethylammonio)-buttersäurebetain Hydrochlorid; 3-(2-benzyloxybenzoyloxy)-4-trimethylammonio)-buttersäurebetain Hydrochlorid; (R)-3-(2-(Butan-2-yloxy)benzoyloxy)-4-trimethylammonio)-buttersäurebetain; (R)-3-(2-Isopropoxybenzoyloxy)-4-trimethylammonio)-buttersäurebetain; (R)-3-(2-benzyloxybenzoyloxy)-4-trimethylammonio)-buttersäurebetain; (S)-3-(2-(Butan-2-yloxy)benzoyloxy)-4-trimethylammonio)-buttersäurebetain; (S)-3-(2-Isopropoxybenzoyloxy)-4-trimethylammonio)-buttersäurebetain; (S)-3-(2-benzyloxybenzoyloxy)-4-trimethylammonio)-buttersäurebetain; (R)-3-(2-(Butan-2-yloxy)benzoyloxy)-4-trimethylammonio)-buttersäurebetain Hydrochlorid; (R)-3-(2-Isopropoxybenzoyloxy)-4-trimethylammonio)-buttersäurebetain Hydrochlorid; (R)-3-(2-benzyloxybenzoyloxy)-4-trimethylammonio)-buttersäurebetain Hydrochlorid; (S)-3-(2-(Butan-2-yloxy)benzoyloxy)-4-trimethylammonio)-buttersäurebetain Hydrochlorid; (S)-3-(2-Isopropoxybenzoyloxy)-4-trimethylammonio)-buttersäurebetain Hydrochlorid und (S)-3-(2-benzyloxybenzoyloxy)-4-trimethylammonio)-buttersäurebetain Hydrochlorid.

BEISPIELE

Die Erfindung wird in den folgenden nicht-limitierenden Beispielen weiter erläutert.

BEISPIEL 1

**(R)-(-)-3-(2-Hydroxybenzoyloxy)-4-trimethylammonio)-buttersäure-Hydrochlorid**

Zu einer Mischung aus 12.0 g Monochloressigsäure, 6.4 g Dichloresssigsäure und 3.0 g L-Carnitin-Hydrochlorid wurden bei 20-25°C innerhalb von 10 Minuten mit 8.5 g 2-Benzyloxybenzoylchlorid getropft. Die Reaktionsmischung wurde 15 Stunden bei 20-25°C gerührt und anschliessend mit 120 ml Diethylether verdünnt. Das ausgefallene Produkt wurde abgenutscht und im Vakuum bei 20-25°C getrocknet.

Ausbeute: 6.0 g
Smp.: 184-185°C (nach Umkristallisation aus Ethanol / Ethylacetat)

BEISPIEL 2

**(R)-(-)-3-(2-Hydroxybenzoyloxy)-4-trimethylammonio)-buttersäure-Hydrochlorid**

In 50.0 g Dichloressigsäure wurden bei 60-65°C 9.9 g L-Carnitin-Hydrochlorid gelöst. Zu dieser Lösung wurden bei -10 bis 0°C innerhalb von 60 Minuten 20.9 g 2-Isopropoxybenzoylchlorid zugetropft. Während des Zutropfens und der folgenden Nachreaktion bei -10 bis 0°C wurde durch Anlegen eines schwachen Vakuums (100-200 mbar) das freiwerdende Chlorwasserstoff-Gas aus der Reaktionsmischung vertrieben. Die Reaktion wurde mittels DC verfolgt (Kieselgel; Laufmittel: Chloroform:Methanol:Wasser:5%ige Ammoniaklösung 55:39:1:5). Nach ca. 1 Stunde wurden 9.4 g Salzsäure-Gas in die Reaktionslösung eingeleitet. Die Reaktionslösung wurde 30 Minuten auf 45-50°C erhitzt, mit geringen Mengen an Wasser versetzt und bei 20 bis 25°C weiter gerührt. Die Reaktion wurde wiederum mittels DC verfolgt (Kieselgel; Laufmittel: Chloroform:Methanol:Wasser:5%ige Ammoniaklösung 55:39:1:5). Anschliessend wurde die Reaktionsmischung mit 200 ml Essigsäureethylester verdünnt und mit Salicyloyl-L-Carnitin-HCl angeimpft. Sobald das Produkt anfing zu kristallisieren, wurden weitere 200 Im Essigsäureethylester hinzugefügt. Die entstandene Suspension wurde auf 10 bis 15°C abgekühlt und noch eine Stunde bei dieser Temperatur gerührt. Das auskristallisierte Produkt wurde abgenutscht, mit 100 ml Essigsäureethylester gewaschen und im Vakuumtrockenschrank bei 40-45°C über Nacht getrocknet.

Ausbeute: 14.6 g (92%)
Smp. : 184-185°C
RF: 0.19 (Kieselgel; Laufmittel: Chloroform:Methanol:Wasser: 5%ige NH3lösung 55:39:1:5)
$[\alpha]_D^{20} = -25.6°$ (c=1, Wasser)

BEISPIEL 3

**(R)-(-)-3-(2-Isopropoxybenzoyloxy)-4-trimethylammonio)-buttersäure**

In 28.0 g Trifluoressigsäure wurden bei 40-45°C 5.0 g L-Carnitin-Hydrochloride gelöst. Zu dieser Lösung wurden bei 0-20°C innerhalb von 20 Minuten 11 g 2-Isopropoxybenzoylchlorid zugetropft. Während des Zutropfens und der folgenden Nachreaktion bei 0-20°C wurde ein Stickstromstrom durch die Lösung geleitet, um das freiwerdende Chlorwasserstoff-Gas zu vertreiben. Die Reaktion wurde mittels DC verfolgt (Kieselgel; Laufmittel: Chloroform:Methanol:Wasser:5%ige Ammoniaklösung 55:39:1:5). Nach ca. 1 Stunde wurde die Reaktionslösung durch Zugabe von 100 mL tert.-Butylmethylether gequencht. Das ausgefallene Produkt wurde in 10 mL Wasser aufgenommen, über eine Säule mit schwach basischem Ionenaustauscherharz filtriert und im Vakuum eingeengt. Der erhaltene Feststoff wurde aus Acetonitril / Aceton umkristallisiert.

Ausbeute: 5.6 g
$[\alpha]_D^{20} = -14.2°$ (c=1, Wasser)
Smp. : 142-144°C
RF: 0.28 (Kieselgel; Laufmittel: Chloroform:Methanol:Wasser:5%ige NH3lösung 55:39:1:5)
1H-NMR (DMSO-d6, 300 MHz) $\delta$ : 7.61-6.95 (m, 4H)

5.69-5.57 (m, 1H)
4.76-4.62 (m, 1H)
3.89-3.68 (m, 2H)
3.14 (s, 9H)
2.43-2.09 (m, 2H)
1.27 (d, 6H)

MS: 323 (M+1)

BEISPIEL 4

**(R)-(-)-3-(2-(Butan-2-yloxy)benzoyloxy)-4-trimethylammonio)-buttersäure**

In 40.0 g Dichloressigsäure wurden bei 40-45°C 4.0 g L-Carnitin gelöst. Zu dieser Lösung wurden bei 0-20°C innerhalb von 20 Minuten 13.2 g 2-sec.-Butoxybenzoylchlorid zugetropft. Während des Zutropfens und der folgenden Nachreaktion bei 0-20°C wurde ein Stickstromstrom durch die Lösung geleitet, um das freiwerdende Chlorwasserstoff-Gas zu vertreiben. Die Reaktion wurde mittels DC verfolgt (Kieselgel; Laufmittel: Chloroform:Methanol:Wasser:5%ige Ammoniaklösung 55:39:1:5). Nach ca. 1.5 Stunden wurde die Reaktionslösung durch Zugabe von 300 mL tert.-Butylmethylether gequencht. Das ausgefallene Produkt wurde in 15 mL Wasser aufgenommen, über eine Säule mit schwach basischem Ionenaustauscherharz filtriert und im Vakuum eingeengt. Der erhaltene Feststoff wurde aus Acetonitril / Aceton umkristallisiert.

Ausbeute: 3.2 g
$[\alpha]_D^{20} = -22.6°$ (c=1, Wasser)
Smp. : 134-135°C
1H-NMR des Hauptdiastereomers (DMSO-d6, 300 MHz)
$\delta$ : 7.63-6.94 (m, 4H)

5.69-5.57 (m, 1H)
4.53-4.41 (m, 1H)
3.89-3.65 (m, 2H)
3.14 (s, 9H)
2.43-2.09 (m, 2H)
1.70-1.51 (m, 2H)

1.24-1.21 (m, 3H)
0.95-0.89 (m, 3H)
MS: 338 (M+1)

## Patentansprüche

1. Verfahren zur Herstellung von 3-(2-hydroxybenzoyloxy)-4-(trimethylammonio)-buttersäurebetain-Hydrochlorid der Formel (I),

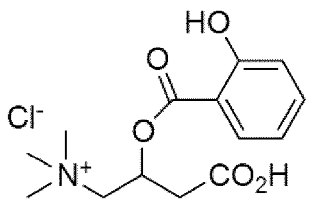

(I)

wobei das Verfahren den Schritt umfasst

(i) Umsetzung von 3-Hydroxy-4-(trimethylammonio)-buttersäurebetain oder einem Salz davon, wobei vorzugsweise das Salz des 3-Hydroxy-4-(trimethylammonio)-buttersäurebetains

das 3-Hydroxy-4-(trimethylammonio)-buttersäurebetain-Hydrochlorid ist, mit einem 2-Alkoxybenzoylchlorid der Formel (III),

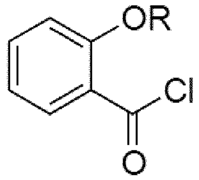

(III)

wobei R ausgewählt ist aus Benzyl und verzweigten C3-C6-Alkylgruppen.

**2.** Verfahren nach Anspruch 1, wobei die besagte Umsetzung (i) umfasst

(1) Anlegen eines Vakuums; oder
(2) Durchleiten eines Inertgases;

und wobei das Verfahren weiter den Schritt umfasst (ii) Versetzen des Reaktionsgemisches der besagten Umsetzung (i) mit Chlorwasserstoffsäure.

**3.** Verfahren nach Anspruch 2, wobei die besagte Umsetzung (i) das Isolieren eines Zwischenproduktes der Formel (II), oder eines Salzes davon, umfasst,

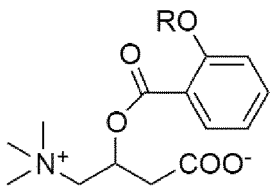

(II)

und das besagte Versetzen des Reaktionsgemisches mit Chlorwasserstoffsäure (ii) das Versetzen des besagten Zwischenproduktes der Formel (II) oder eines Salzes davon mit Chlorwasserstoffsäure ist.

**4.** Verfahren nach einem der Ansprüche 2 oder 3, wobei die besagte Umsetzung (i) das Anlegen eines Vakuums umfasst, und wobei das Vakuum 50 mbar bis 800 mbar beträgt.

**5.** Verfahren nach einem der Ansprüche 1 bis 4, wobei die besagte Umsetzung (i) und das besagte Versetzen des Reaktionsgemisches mit Chlorwasserstoffsäure (ii) in einem Lösungsmittel durchgeführt wird, wobei das Lösungsmittel ein protisches, polares und gegenüber Chlorwasserstoffsäure inertes Lösungsmittel ist.

**6.** Verfahren nach Anspruch 5, wobei das besagte protische, polare und gegenüber Chlorwasserstoffsäure inerte Lösungsmittel aus halogenierten C1-C6-Carbonsäuren und Gemischen davon ausgewählt ist.

**7.** Verfahren nach einem der vorangegangenen Ansprüche, wobei R ausgewählt ist aus Benzyl und sekundären C3-C6-Alkylgruppen.

**8.** Verfahren nach Anspruch 7, wobei R ausgewählt ist aus Benzyl, *iso*-Propyl, *sec*-Butyl, und 2-Pentyl (*sec*-Pentyl).

**9.** Verfahren nach einem der Ansprüche 1 bis 8, wobei die besagte Umsetzung (i) bei einer Temperatur von -20°C bis +30°C durchgeführt wird.

**10.** Verfahren nach einem der Ansprüche 2 bis 9, wobei das besagte Versetzen des Reaktionsgemisches mit Chlorwasserstoffsäure (ii) bei einer Temperatur von 20°C bis 100°C durchgeführt wird.

**11.** Verfahren nach einem der Ansprüche 2 bis 10, wobei das besagte Versetzen des Reaktionsgemisches mit Chlorwasserstoffsäure (ii) unter einem Überdruck gemessen am Umgebungsdruck durchgeführt wird.

**12.** Verfahren nach einem der Ansprüche 1 bis 11, wobei das Verfahren weiter den Schritt umfasst
(iii) Isolieren des 3-(2-hydroxybenzoyloxy)-4-(trimethylammonio)-buttersäurebetain-Hydrochlorid der Formel (I), wobei das Isolieren durch Kristallisation durch Zugabe eines unpolaren Lösungsmittels erfolgt.

**13.** Verfahren nach einem der vorangegangenen Ansprüche, wobei die besagte Umsetzung (i) mit (R)-(-)-3-Hydroxy-4-(trimethylammonio)-buttersäurebetain Hydrochlorid und besagtem 2-Alkoxybenzoylchlorid der Formel (III) erfolgt.

**14.** Verfahren zur Herstellung einer Verbindung der Formel (II), oder ein Salz davon,

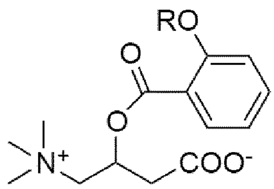

(II)

wobei das Verfahren den Schritt umfasst

(i) Umsetzung von 3-Hydroxy-4-(trimethylammonio)-buttersäurebetain oder einem Salz davon, wobei vorzugsweise das Salz des 3-Hydroxy-4-(trimethylammonio)-buttersäurebetains das 3-Hydroxy-4-(trimethylammonio)-buttersäurebetain-Hydrochlorid ist, mit einem 2-Alkoxybenzoylchlorid der Formel (III),

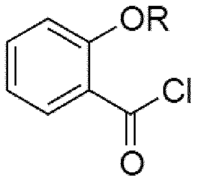

(III)

wobei R ausgewählt ist aus Benzyl und verzweigten C3-C6-Alkylgruppen; und wobei die besagte Umsetzung (i), typisch und bevorzugt zur Entfernung des gebildeten HCl, umfasst

(1) Anlegen eines Vakuums; oder
(2) Durchleiten eines Inertgases.

**15.** Verbindung der Formel (II), oder ein Salz davon,

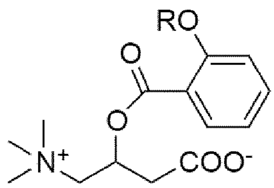

(II)

wobei R ausgewählt ist aus Benzyl und verzweigten C3-C6-Alkylgruppen.

**Claims**

**1.** Method for the preparation of 3-(2-hydroxybenzoyloxy)-4-(trimethylammonio)-butyric acid betaine hydrochloride of formula (I),

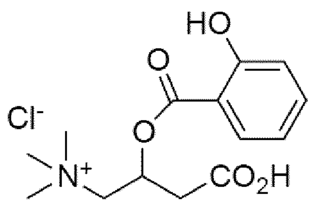

(I)

wherein the method comprises the step of

(i) reacting 3-hydroxy-4-(trimethylammonio)-butyric acid betaine or a salt thereof, the salt of 3-hydroxy-4-(trimethylammonio)-butyric acid betaine preferably being 3-hydroxy-4-(trimethylammonio)-butyric acid betaine hydrochloride, with a 2-alkoxybenzoyl chloride of formula (III),

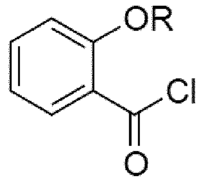

(III)

wherein R is selected from benzyl and branched C3-C6 alkyl groups.

**2.** Method according to claim 1, wherein said reaction (i) comprises

(1) applying a vacuum; or
(2) passing an inert gas;

and wherein the method further comprises the step of
(ii) adding hydrochloric acid to the reaction mixture of said reaction (i).

**3.** Method according to claim 2, wherein said reaction (i) comprises isolating an intermediate product of formula (II), or a salt thereof,

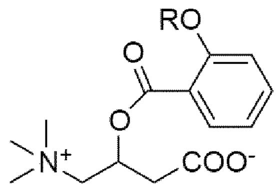

(II)

and said addition of hydrochloric acid to the reaction mixture (ii) is the addition of hydrochloric acid to said intermediate product of formula (II) or a salt thereof.

**4.** Method according to either claim 2 or claim 3, wherein said reaction (i) comprises applying a vacuum, and wherein the vacuum is 50 mbar to 800 mbar.

**5.** Method according to any of claims 1 to 4, wherein said reaction (i) and said addition of hydrochloric acid to the reaction mixture (ii) are carried out in a solvent, wherein the solvent is a protic, polar solvent that is inert with respect to hydrochloric acid.

**6.** Method according to claim 5, wherein said protic, polar solvent that is inert with respect to hydrochloric acid is selected from halogenated C1-C6 carboxylic acids and mixtures thereof.

**7.** Method according to any of the preceding claims,

wherein R is selected from benzyl and secondary C3-C6 alkyl groups.

**8.** Method according to claim 7, wherein R is selected from benzyl, *iso*-propyl, sec-butyl, and 2-pentyl (sec-pentyl).

**9.** Method according to any of claims 1 to 8, wherein said reaction (i) is carried out at a temperature of-20°C to +30°C.

**10.** Method according to any of claims 2 to 9, wherein said addition of hydrochloric acid to the reaction mixture (ii) is carried out at a temperature of 20°C to 100°C.

**11.** Method according to any of claims 2 to 10, wherein said addition of hydrochloric acid to the reaction mixture (ii) is carried out under an overpressure measured at ambient pressure.

**12.** Method according to any of claims 1 to 11, the method further comprising the step of (iii) isolating the 3-(2-hydroxybenzoyloxy)-4-(trimethylammonio)-butyric acid betaine hydrochloride of formula (I), wherein the isolation is carried out by crystallization by adding a non-polar solvent.

**13.** Method according to any of the preceding claims, wherein said reaction (i) takes place with (R)-(-)-3-hydroxy-4-(trimethylammonio)-butyric acid betaine hydrochloride and said 2-alkoxybenzoyl chloride of formula (III).

**14.** Method for preparing a compound of formula (II), or a salt thereof,

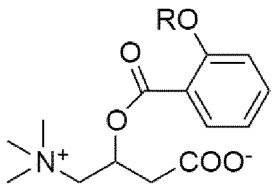

(II)

wherein the method comprises the step of

(i) reacting 3-hydroxy-4-(trimethylammonio)-butyric acid betaine or a salt thereof, the salt of 3-hydroxy-4-(trimethylammonio)-butyric acid betaine preferably being 3-hydroxy-4-(trimethylammonio)-butyric acid betaine hydrochloride, with a 2-alkoxybenzoyl chloride of formula (III),

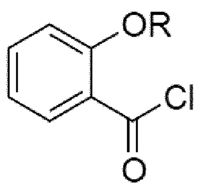

(III)

wherein R is selected from benzyl and branched C3-C6 alkyl groups; and wherein said reaction (i), typically and preferably for removing the HCl formed, comprises

(1) applying a vacuum; or
(2) passing an inert gas.

**15.** Compound of formula (II), or a salt thereof,

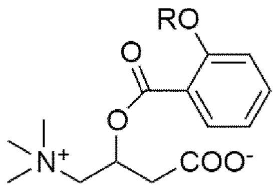

(II)

wherein R is selected from benzyl and branched C3-C6 alkyl groups.

**Revendications**

**1.** Procédé de préparation de chlorhydrate de bétaïne d'acide 3-(2-hydroxybenzoyloxy)-4-(triméthylammonio)-butyrique de formule (I),

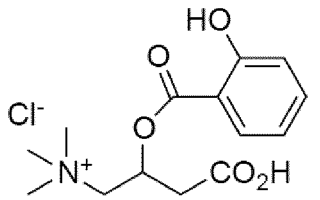

(I)

le procédé comprenant l'étape

(i) de réaction de la bétaïne d'acide 3-hydroxy-4-(triméthylammonio)-butyrique ou d'un sel de celle-ci, où le sel de la bétaïne d'acide 3-hydroxy-4-(triméthylammonio)-butyrique est de préférence le chlorhydrate de bétaïne d'acide 3-hydroxy-4-(triméthylammonio)-butyrique, avec un chlorure de 2-alcoxybenzoyle de formule (III),

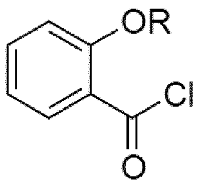

(III)

où R est choisi entre les groupes benzyle et alkyle ramifié en C3-C6.

**2.** Procédé selon la revendication 1, où ladite réaction (i) comprend

(1) la création d'un vide ; ou
(2) le passage d'un gaz inerte ;

et où le procédé comprend en outre l'étape
(ii) d'ajout d'acide chlorhydrique au mélange réactionnel de ladite réaction (i).

**3.** Procédé selon la revendication 2, où ladite réaction (i) comprend l'isolement d'un produit intermédiaire de formule (II), ou d'un sel de celui-ci,

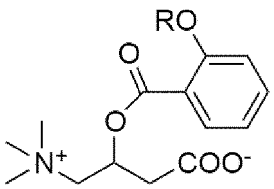

(II)

et ledit ajout d'acide chlorhydrique au mélange réactionnel (ii) est un ajout d'acide chlorhydrique audit produit intermédiaire de formule (II) ou à un sel de celui-ci.

**4.** Procédé selon l'une des revendications 2 ou 3, où ladite réaction (i) comprend la création d'un vide, et où le vide est de 50 mbar à 800 mbar.

**5.** Procédé selon l'une quelconque des revendications 1 à 4, où ladite réaction (i) et ledit ajout d'acide chlorhydrique au mélange réactionnel (ii) sont effectués dans un solvant, où le solvant est un solvant protique, polaire et inerte par rapport à l'acide chlorhydrique.

**6.** Procédé selon la revendication 5, où ledit solvant protique, polaire et inerte par rapport à l'acide chlorhydrique est choisi entre les acides carboxyliques halogénés en C1-C6 et les mélanges de ceux-ci.

**7.** Procédé selon l'une quelconque des revendications précédentes, où R est choisi entre les groupes benzyle et alkyle secondaire en C3-C6.

**8.** Procédé selon la revendication 7, où R est choisi parmi le benzyle, l'*iso*-propyle, le *sec*-butyle et le 2-pentyle (*sec*-pentyle).

**9.** Procédé selon l'une quelconque des revendications 1 à 8, où ladite réaction (i) est effectuée à une température comprise entre -20 °C et +30 °C.

**10.** Procédé selon l'une quelconque des revendications 2 à 9, où ledit ajout d'acide chlorhydrique au mélange réactionnel (ii) est effectué à une température comprise entre 20 °C et 100 °C.

**11.** Procédé selon l'une quelconque des revendications 2 à 10, où ledit ajout d'acide chlorhydrique au mélange réactionnel (ii) est effectué sous une surpression mesurée à pression ambiante.

**12.** Procédé selon l'une quelconque des revendications 1 à 11, où le procédé comprend en outre l'étape
(iii) d'isolement du chlorhydrate de bétaïne d'acide 3-(2-hydroxybenzoyloxy)-4-(triméthylammonio)-butyrique de formule (I), où l'isolement est réalisé par cristallisation par addition d'un solvant apolaire.

**13.** Procédé selon l'une quelconque des revendications précédentes, où ladite réaction (i) est réalisée avec le chlorhydrate de bétaïne d'acide (R)-(-)-3-hydroxy-4-(triméthylammonio)-butyrique et ledit chlorure de 2-alcoxybenzoyle de formule (III).

**14.** Procédé de préparation d'un composé de formule (II), ou d'un sel de celui-ci,

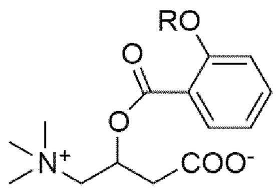

(II)

le procédé comprenant l'étape

(i) de réaction de la bétaïne d'acide 3-hydroxy-4-(triméthylammonio)-butyrique ou d'un sel de celle-ci, où le sel de la bétaïne d'acide 3-hydroxy-4-(triméthylammonio)-butyrique est de préférence le chlorhydrate de bétaïne d'acide 3-hydroxy-4-(triméthylammonio)-butyrique, avec un chlorure de 2-alcoxybenzoyle de formule (III),

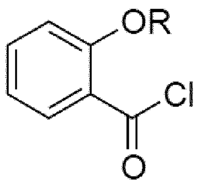

(III)

où R est choisi entre les groupes benzyle et alkyle ramifié en C3-C6 ; et où ladite réaction (i) comprend, typiquement et de préférence pour éliminer le HCl formé

(1) la création d'un vide ; ou
(2) le passage d'un gaz inerte.

**15.** Composé de formule (II), ou un sel de celui-ci,

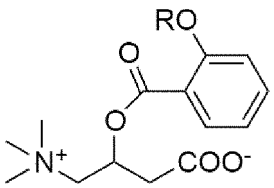

(II)

où R est choisi entre les groupes benzyle et alkyle ramifié en C3-C6.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 553385 A


### In der Beschreibung aufgeführte Nicht-Patentliteratur


- **ALAN R. KATRITZKY ; DAN C. FARA ; HONG-FANG YANG ; KAIDO TÄMM et al.** Spectroscopic Measurements. *Quantitative Measures of Solvent Polarity,* 183